# EUROPEAN PATENT SPECIFICATION

(11) **EP 4 231 891 B1**
(45) Date of publication and mention of the grant of the patent: **25.12.2024**
(21) Application number: 21801845.5
(22) Date of filing: 20.10.2021
(51) Int. Cl.: A61B 1/00, A61B 1/12, A61M 1/00, A61M 3/02

(54) **A CONNECTOR TO FLUIDICALLY CONNECT A MEDICAL DEVICE WITH AIR AND WATER TUBES**
VERBINDER ZUM FLUIDISCHEN VERBINDEN EINER MEDIZINISCHEN VORRICHTUNG MIT LUFT- UND WASSERROHREN
RACCORD DE RACCORDEMENT FLUIDIQUE D'UN DISPOSITIF MÉDICAL AVEC TUBES D'AIR ET D'EAU

(30) Priority: 20.10.2020 EP 20202925
(43) Date of publication of application: 30.08.2023
(73) Proprietor: GA Health Medical Devices Limited, Mullingar Co. Westmeath (IE)
(72) Inventor: CHAU, Steven, ShekMun Shatin, N.T. (HK)
(74) Representative: Purdylucey Intellectual Property
(86) International application number: PCT/EP2021/079142
(87) International publication number: WO 2022/084418

(56) References cited:
- AU-B2- 2011 316 038
- US-A- 5 297 537
- US-A9- 2018 206 707

## Description

### Field of the Invention

The present invention relates to a connector to fluidically connect a medical device with air and water tubes. In particular, the invention relates to a connector to fluidically connect an endoscope with air and water tubing leading to a water bottle

### Background to the Invention

Endoscopes are imaging devices used to image the inside of the body. They generally have a control handle coupled to a long elongated flexible tubular body with an imaging lens and light at a distal end. Optical fibres are often employed to relay light from an illumination source in the handle to the distal tip of the elongated body. Most endoscopes incorporate air/water channels to deliver air and water to the distal tip for the purpose of cleaning the lens (water) or insufflating tissue close to the tip. The air is generally provided by a pump that is connected to an air line in the handle of the endoscope. Water is provided by an external water bottle configured for connection to the handle of the endoscope by a water line and pump. Irrigation and rinsing tube sets and systems are described in AU2011316038, US5297537 and US20180206707.

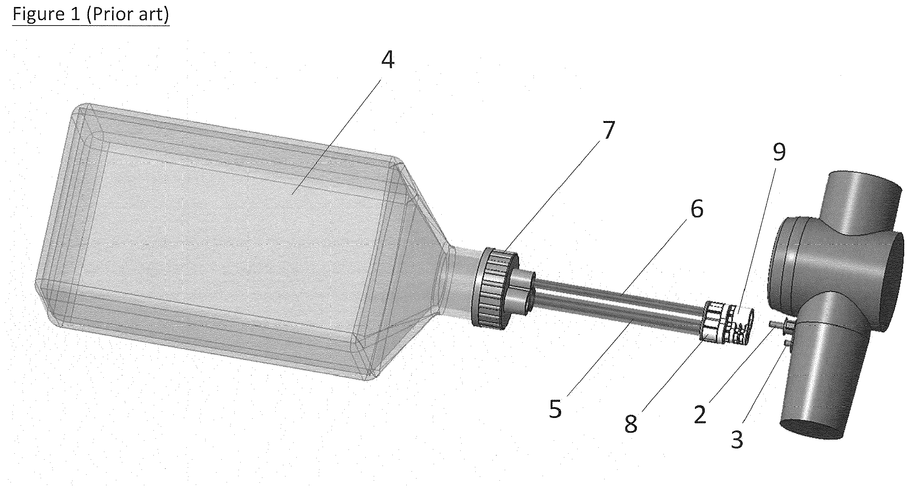

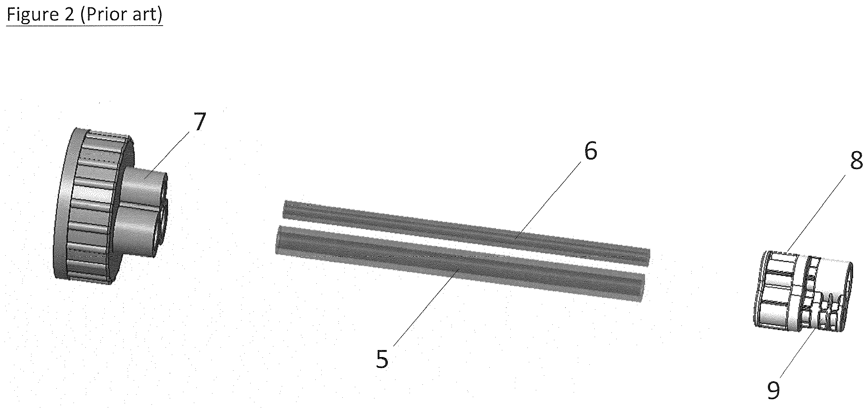

In some endoscopes, air can be routed by means of a valve to an external water bottle to pressurise the bottle. In this type of endoscope, the handle has adjacent air and water ports provided on the handle to fluidically connect a water bottle with the endoscope by means of air and water tubes. In use, air is pumped from the endoscope to the water bottle via an air tube to pressurise the bottle, and the pressure forces water in the bottle into the endoscope through the water line, where the water is used to clean the lens of the endoscope. Figures 1 and 2 illustrate this type of system, with a handle of an endoscope 1 with protruding water port 2 and air port 3, water bottle 4, air and water tubes 5, 6, bottle cap 7 glued to a distal end of the air and water tubes, and a connector 8 glued to a distal end of the air and water tubes and having a distal end 9 dimensioned to mate the fluidically connect with the water port 2 and air port 3. Figure 2 shows the connector 8, tubes 5, 6 and bottle cap 7 prior to assembly.

The water bottle, tubes and connector are generally used for a 20-hour period before being disposed of. This will mean that they are used on several different patients during this period. While none of these components come into contact with the patient, there is a risk of back-flow (reflux) of water from the endoscope into the water bottle during use. This can cause contamination of water in the water bottle, which can lead to cross-contamination when the endoscope is used on a different patient using the same water bottle and tubing. This problem can be addressed by changing the whole water supply assembly after every patient, although this is an expensive solution as well as being environmentally unfriendly.

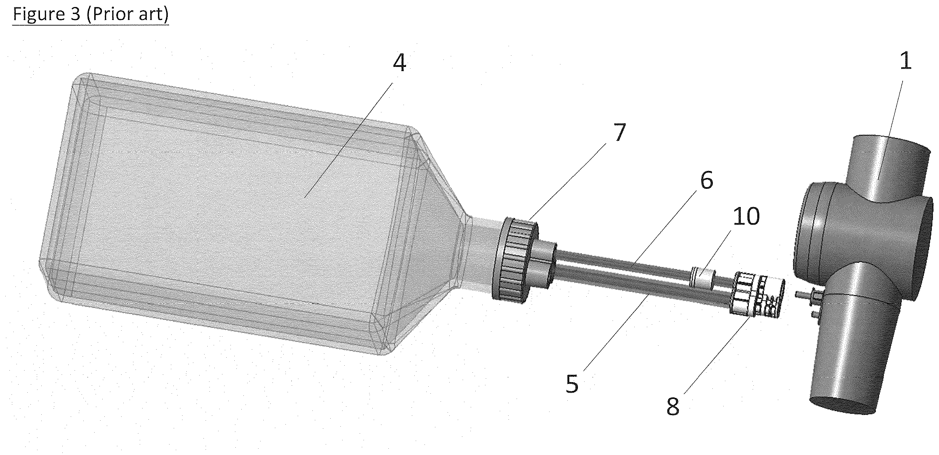

To address this problem, it is recommended to incorporate a non-return valve into the fluidic conduits to prevent reflux of water into the bottle. Incorporating a non-return valve into existing machines is expensive and time consuming. An alternative solution is to incorporate a non-return valve into the water line as illustrated in Figure 3 in which the non-return valve 10 is provided in the water tube 6. However, while this will prevent reflux water into the bottle allowing the bottle to be re-used, the rest of the water supply assembly (cap, tubing, and connector) all must be disposed of because they are glued together and cannot be separated from the potentially contaminated connector. Again, this is an expensive solution.

It is an object of the invention to overcome at least one of the above-referenced problems.

### Summary of the Invention

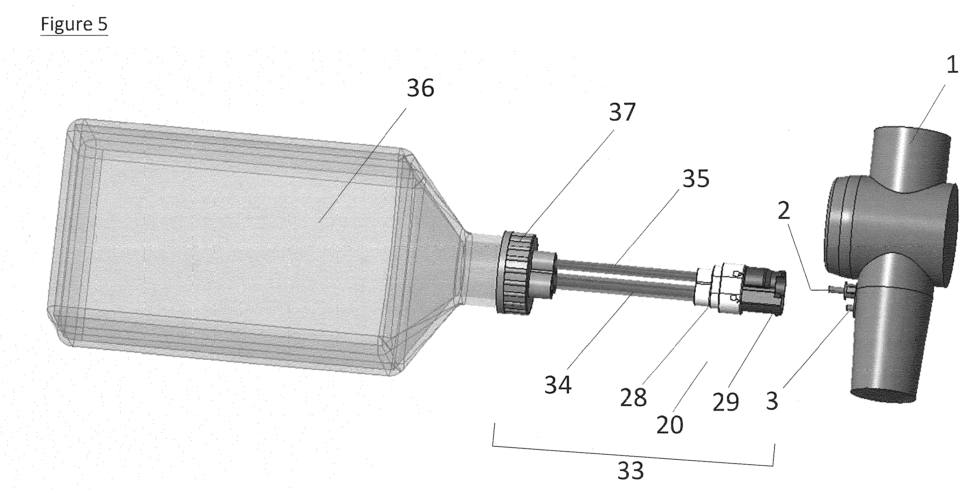

The Applicant has addressed the problems of the prior art by providing a modified connector, that is provided in a two-part form with a distal part detachably attached to a proximal part and where the water lumen of the distal part incorporates a one-way valve. This is illustrated in an exploded view in Figure 5 showing the detachable distal and proximal parts and the non-return valve in the distal part. With this connector, after the endoscope has been used with a patient, the connector is detached from the air and water ports of the endoscope, and the distal part of the connector is detached from the proximal part, and a replacement distal part is attached to the proximal part to re-assemble the connector, which is then reattached to the ports of the endoscope. In this way, only the distal part of the connector must be discarded and the remaining parts (proximal part of the connector, attached water and air tubes, bottle cap and bottle) may be re-used for the remainder of the recommended 20-hour period. This allows a kit to be supplied comprising a water bottle, a tubing assembly with bottle cap, air and water tubes, and a connector, and several replacement distal parts for the connector, that can be used with several patients during a 20-hour period without any risk of cross-contamination.

In a first aspect, the invention provides a connector to fluidically connect a medical instrument with an air tube and a water tube, the connector comprising a housing having:
a first through lumen with a proximal opening configured to couple with an air tube and a distal opening configured to couple with an air port of the medical instrument; and
a second through lumen with a proximal opening configured to couple with a water tube and a distal opening configured to couple with a water port of the medical instrument;
characterised in that the connector is a two-part connector comprising a proximal part comprising the proximal openings and a distal part comprising the distal openings, wherein the proximal part and distal part are configured for detachable engagement, and wherein the second through lumen comprises a non-return valve disposed in the distal part of the housing.

In any embodiment, the distal openings are configured to fluidically connect with air and water ports of a scoping device. Typically, the ports are protruding ports.

In any embodiment, the distal openings are configured to fluidically connect with an air-water (AW) port of an endoscope.

In any embodiment, each proximal opening comprises a recessed socket dimensioned to receive an end of an air or water tube.

In any embodiment, the recessed socket of the first through lumen has a diameter greater than the recessed socket of the second through lumen.

In any embodiment, the proximal part and distal part are configured for detachable friction-fit engagement.

In any embodiment, a proximal end of the distal part of the housing comprises projecting ports, and a distal end of the proximal part of the housing comprises recessed ports configured to receive the projecting ports to fluidically couple the distal and proximal parts of the housing.

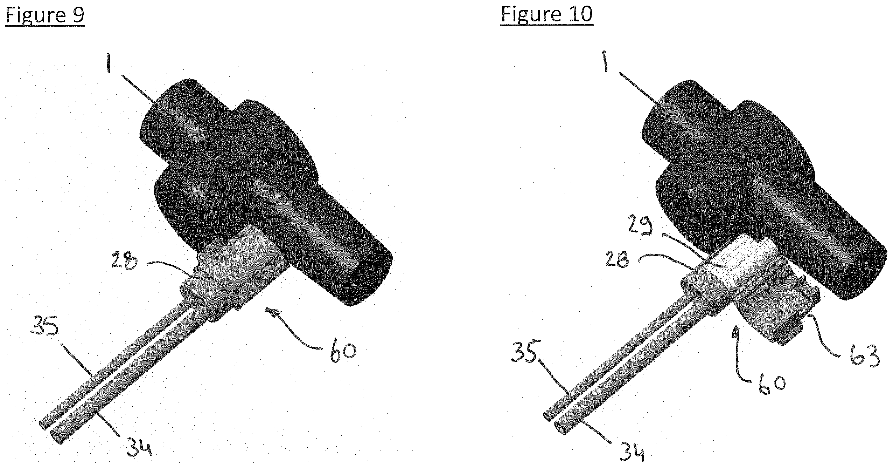

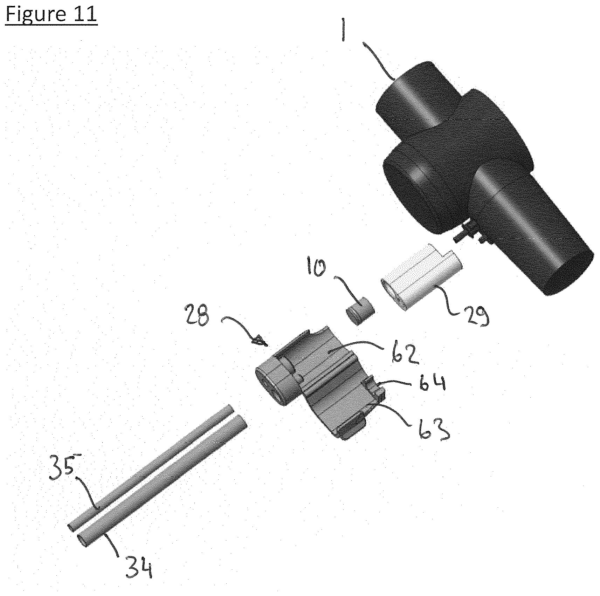

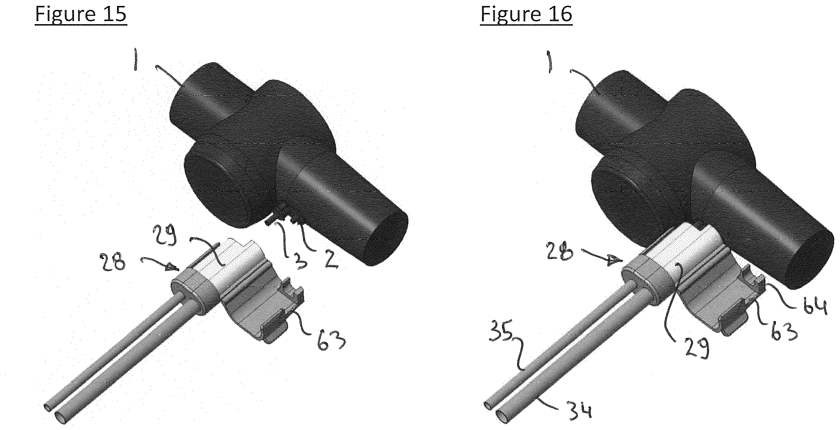

In any embodiment, the proximal part comprises a housing section dimensioned to receive the distal part. An example of this embodiment is illustrated in Figures 9 to 18.

In any embodiment, the housing section of the proximal part comprises a base section and a lid section hingedly mounted to the base section and adjustable from an open configuration to allow insertion of the distal part into the housing section and a closed configuration in which the distal part is enclosed within the housing section. The base section may be dimensioned to allow all, substantially all, or part of the distal part of the housing nest within the base when the lid is open.

In any embodiment, the lid section comprises a clamp configured to clamp the proximal part of the housing to the air port or water port of the medical instrument when the lid section is in the closed configuration. The clamp may be a C-shaped element configured to snap fit to the air or water port.

In any embodiment, the lid section comprises a clamp configured to clamp the proximal part of the housing to the air port of the medical instrument when the lid section is in the closed configuration.

In any embodiment, the distal opening of the second through lumen is recessed proximally relative to the distal opening of the first through lumen.

In any embodiment, the connector consists essentially of a proximal part comprising the housing section and the lid section, a distal part, and a non-return valve. In this embodiment, the distal part and non-return valve are disposed and detached and discarded after every use, and the proximal part may be re-used and only replaced after a defined period of use, for example 24 hours.

The invention also provides an air and water tube assembly comprising:
a connector according to the invention;
a water tube having a distal end fluidically coupled to the first through lumen of the connector via the proximal opening; and
an air tube having a distal end fluidically coupled to the second through lumen of the connector via the proximal opening.

In any embodiment, the distal ends of the air and water tubes are non-detachably attached to the connector (e.g. glued).

In any embodiment, the assembly comprises a bottle cap non-detachably connected to a proximal part of the water tube and air tube to fluidically connect a bottle with the air and water tubes when the cap is attached to the bottle. The cap generally includes bores for receipt of the distal part of the water tune and air tube. Generally, the water tube is fixed to the cap such that a distal part of the tube extends beyond the cap (allowing the tube project into the bottle and into the water in the bottle when the cap is fitted to the bottle).

In any embodiment, the water tube has a bore that is smaller than a bore of the second tube.

The invention also provides a kit of parts comprising an assembly of the invention, and additionally including one or more replacement distal parts of the connector configured for detachable attachment to the proximal part of the connector.

In any embodiment, the medical device is an endoscope of the type having adjacent air and water ports on the handle of the endoscope. In any embodiment, the endoscope is an Olympus 025 endoscope. Other endoscopes suitable for use with the connector, assembly and methods of the invention include the Fuji 088, Fuji 090 and Pentax 072/076 endoscopes.

An additional and related aspect of the invention is an adapter for a connector of an air and water tube assembly, that allows connectors of conventional air and water tube assemblies (e.g. as shown in Figures 1 and 2) connect with an air and water port of a medical instrument such as a scoping device (e.g. an endoscope or the like) while allowing re-use of the air and water assembly with multiple patients. The adapter comprises a housing having:
a distal end with an air port socket dimensioned to mate with the air port of the medical instrument and a water port socket dimensioned to mate with the water port of the medical instrument;
a proximal end with an air port and a water port with the same configuration as the air and water ports of the medical instrument;
an air lumen fluidically connecting the air port socket and the air port;
a water lumen fluidically connecting the water port socket and water port; and
a non-return valve disposed in the water lumen.

Typically, the air and water port sockets of the distal end of the adapter are configured to fluidically connect with air and water ports of an endoscope, typically an Olympus 025, Fuji 088, Fuji 090 or Pentax 072/076 endoscopes.

Other aspects and preferred embodiments of the invention are defined and described in the other claims set out below.

### Brief Description of the Figures

**FIG. 1** (Prior art) is an illustration of a handle of an endoscope, a water bottle, and known a water and air tube assembly for fluidically connecting the water bottle and the endoscope.
**FIG.2** (Prior art) is an illustration of the water and air tube assembly of FIG. 1 in an exploded form.
**FIG. 3** (Prior art) is an illustration of a handle of an endoscope, a water bottle, and a water and air tube assembly for fluidically connecting the water bottle and the endoscope, with a non-return valve in the water tube.
**FIG. 4A** is a sectional view of a connector of the invention showing the distal and proximal parts prior to engagement.
**FIG. 4B** is an illustration of the connector of FIG. 4A with the distal and proximal parts attached together.
**FIG. 5** is an illustration of an air and water tube assembly of the invention with the cap attached to a water bottle and showing the connector approaching the air and water port on the handle of an endoscope.
**FIG.6** is an exploded sectional view of the water and air tube assembly of FIG. 5 in showing the two-part connector in a detached configuration.
**FIG. 7** is an illustration of an air and water port of an Olympus 025 endoscope.
**FIG. 8** an illustration of a modular adapter for a connector of an air and water tube assembly, that allows connectors of conventional air and water tube assemblies as shown in Figures 1 and 2 connect with an air and water port of a scoping device while allowing the air and water assembly with multiple patients.
**FIG. 9** is an illustration of a connector according to an alternative embodiment of the invention in which the distal part of the housing is contained within a housing section of the proximal part of the housing.
**FIG. 10** is an illustration of the connector of Figure 9 showing the lid section open and the distal part of the housing nested within the housing section of the proximal part.
**FIG. 11** is an exploded view of an air and water tube assembly of the invention that incorporates a connector of Figures 9 and 10.
**FIG. 12** illustrates the connector of Figures 9 and 10 attached to water and air tubes with the lid section open showing the base section and a clamp on an underside of the lid section.
**FIG. 13** is an exploded view of the distal part of the connector and one-way valve prior to being placed within the distal part.
**FIG. 14** is an exploded view of the connector showing how the distal part is nested within the housing section of the proximal part.
**FIG. 15** illustrates the connector of Figures 9 to 14 attached to water tubes with the lid section open and prior to attachment of air and water ports of an endoscope.
**FIG. 16** illustrates the connector attached to the air and water ports of the endoscope and prior to the lid section being closed.
**FIGS. 17 and 18** illustrate how the connector is clamped to air port when the lid section of the housing is closed.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

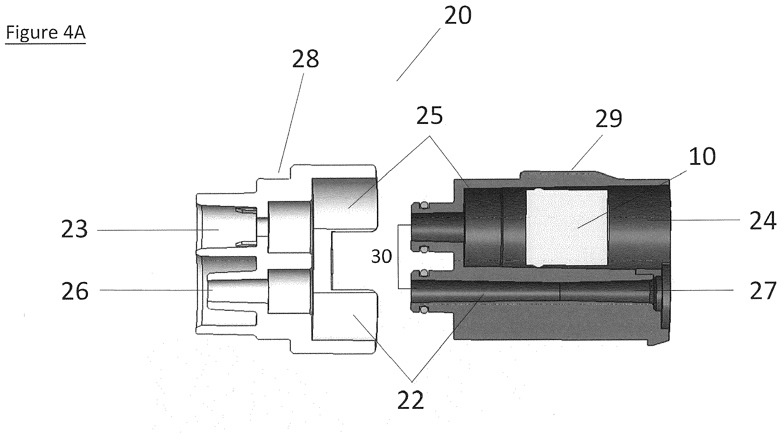

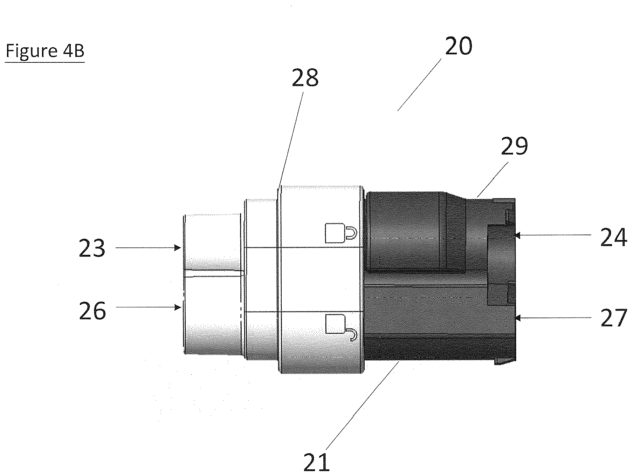

Referring to the drawings and initially to Figures 4A and 4B, there is illustrated a connector to fluidically connect a medical instrument with an air tube and a water tube according to the invention, indicated generally by the reference numeral 20. The connector 20 comprises a housing 21 having a first through lumen 22 for air having a proximal opening 23 and a distal opening 24, and a second through lumen 25 for water having a proximal opening 26 and a distal opening 27. The housing 21 has a proximal part 28 comprising the proximal openings 23, 26 configured for detachable engagement with a distal part 29 comprising the distal openings 24, 27. The second through lumen 25 has a non-return valve 10 disposed in the distal part of the housing that in use prevents back-flow of water from the endoscope to the water bottle and tubing.

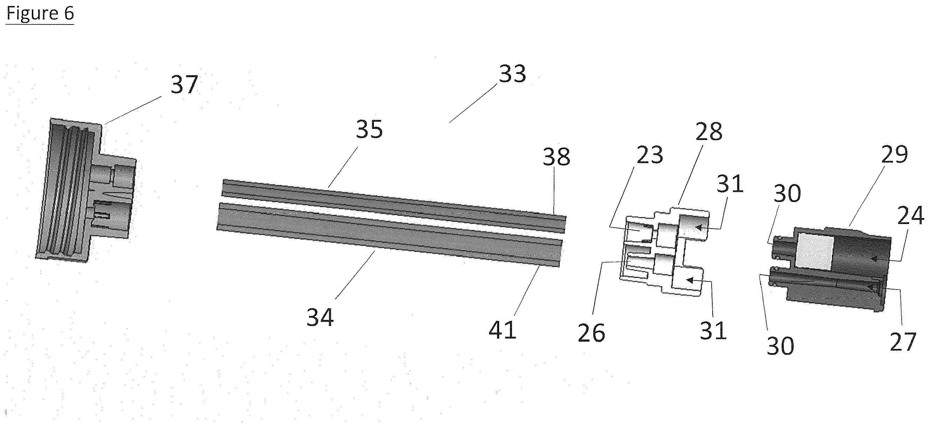

In more detail and referring to Figure 6, a proximal end of the distal part 29 of the housing has projecting outlet ports 30 configured for friction fit engagement with corresponding sockets 31 disposed on a distal end of the proximal part 28 of the housing, to fluidically couple the distal part 29 and proximal part 28 together.

Referring to Figures 5 and 6, there is illustrated an air and water assembly according to the invention 33 and comprising a two-part connector 20, air tube 34, water tube 35, and bottle cap 37. The bottle cap 37 is shown attached to a water bottle 36. The air tube 33 has a distal end 38 fluidically connected to the first through lumen 21 of the connector 20 and a proximal end 39 connected to a through hole 40 in the bottle cap 37 for fluidic connection with the bottle 36. The water tube 35 has a distal end 41 fluidically connected to the second through lumen 25 of the connector 20 and a proximal end 42 connected to a through hole 43 in the bottle cap 37 for fluidic connection with the water bottle 36 for pressurising the bottle. Although not shown, the proximal end 42 of the water tube 35 extends into the bottle to that an inlet of the water tube is under the level of the water in the bottle during use. It will be appreciated that the air and water tubes illustrated are quite short but in practice they will be much longer.

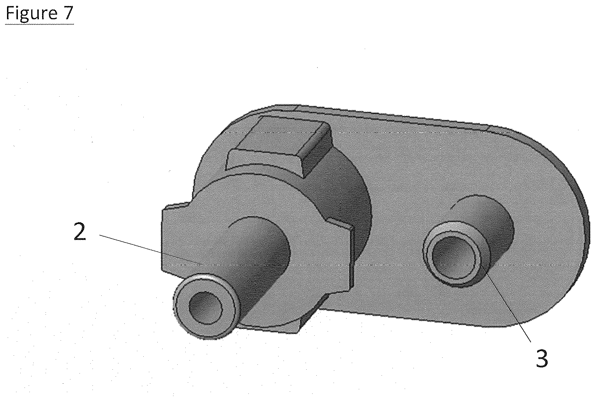

Figure 5 also shown the handle 1 of an endoscope with air port 3 and water port 2. Figure 7 shows the air and water ports 3, 2 in more detail.

In use, and referring to Figure 5, the air and water assembly 33 is used to couple the air and water ports 3, 2 of the endoscope handle 1 with a water bottle 36. The bottle 36 is filled with water and the bottle cap 37 is then attached to the bottle to seal the bottle. During use of the endoscope, pressurised air is provided to the endoscope from an external pump, and upon actuation the pressurised air may be diverted to the water bottle through the connector 20 and air tube 34 to pressurise the bottle. Upon pressurisation, water in the bottle is forced into the endoscope through the water tube 35 and connector 20 where the water is routed by internal tubing a distal end of the endoscope for the purpose of cleaning the lens. The flow of water can be stopped by diverting the flow of pressurised air to the bottle through the air tube 34. During the procedure, any backflow (reflux) of water into the tubing and bottle is prevented by the non-return valve in the distal part of the connector, ensuring that the proximal part of the connector, tubing and water bottle remain sterile. After the endoscopy procedure in a patient is finished, the connector 20 is detached from the handle of the endoscope and the endoscope may then be cleaned and sterilised. To prepare the air and water assembly for the next patient, and to ensure that the tubing and bottle and water is not contaminated with biological matter from the first patient, the distal part 29 of the connector 20 (containing the non-return valve in the second through lumen) is detached from the proximal part 28 and discarded, and a replacement distal part is attached to the proximal part. The connector is then attached to the air and water ports of the sterilised endoscope, and an endoscopy procedure may be carried out on the next patient without any risk of cross-contamination. In this way, the use of the two-part connector with a non-return valve in the water lumen of the distal part allows the bottle, water, tubing, and proximal part of the connector to be re-used numerous times during a 20-hour period, with only the distal part of the connecter being discarded after every patient.

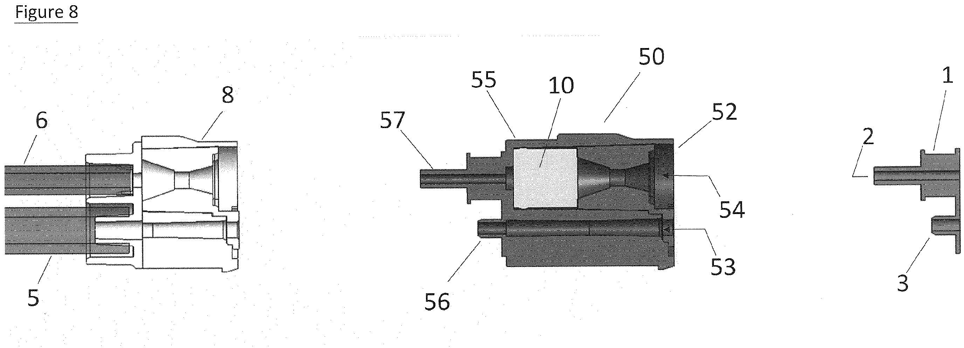

Figure 8 illustrates an adapter fitting according to the invention, indicated generally by the reference numeral 50, in which parts described with reference to the previous embodiments are assigned the same reference numerals. The adapter fitting 50 is designed to be used with an existing air and water assembly such as that illustrated in Figures 1 and 2 and is used to convert a conventional one-part connector into a modified two-part connector with a non-return valve. The adapter 50 comprises a housing 51 having a distal end 52 with an air port socket 53 dimensioned to mate with the air port 3 of an endoscope 1 and a water port socket 54 dimensioned to mate with the water port 2 of the endoscope. A proximal end 55 comprises an air port 56 and a water port 57 identical to the air and water ports of the endoscope (in this case an Olympus 025 endoscope) and configured to fluidically couple with air and water sockets of the conventional connector 8. The adapter comprises an air lumen 58 fluidically connecting the air port socket 53 and the air port 56 and a water lumen 59 fluidically connecting the water port socket 54 and water port 57. A non-return valve 10 is disposed in the water lumen 59 to prevent backflow of water through the adapter.

In use, the adapter 50 may be coupled to a connector of a conventional air and water tube assembly, and then attached to the air and water port of an endoscope. Upon completion of the endoscopy in a first patient, the adapter is detached from the connector and discarded, and a replacement adapter attached to the connector before use in an endoscopy procedure in a second patient. The adapter functions in the same way as the distal part of the two-part connector, ensuring that if any backflow of water occurs that it does not come into contact with the connector allowing the connector and downstream tubing and bottle to be re-used.

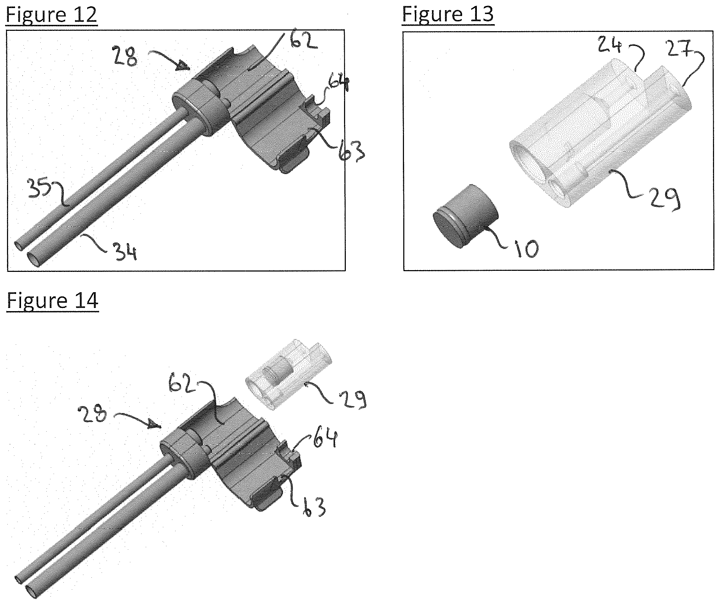

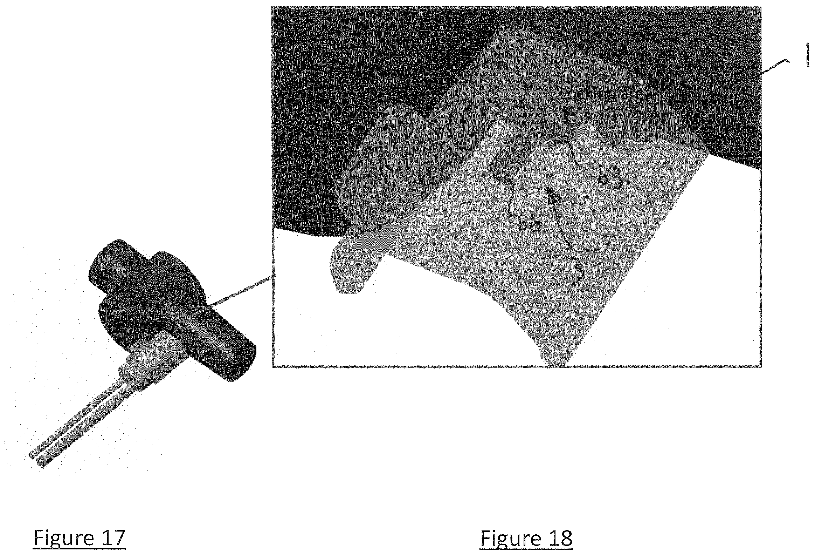

Figures 9 to 18 illustrate a connector according to an alternative embodiment of the invention, indicated generally by the reference numeral 60, in which parts described with reference to the previous embodiments are assigned the same reference numerals. In this embodiment, the proximal part 28 of the connector has a housing section 61 that is dimensioned to the distal part 29 of the connector is a nested arrangement. The housing section has a base section 62 and a lid section 63 hingedly mounted to the base section and adjustable from an open configuration to allow insertion of the distal part 29 into the housing section and a closed configuration in which the distal part 29 is enclosed within the housing section. The base section and lid section are dimensioned to receive and enclose the distal part of the connector when the lid section is closed, with a distal end of the housing section open to allow the distal part of the connector fluidically couple to the air and water ports of the endoscope. The distal part 29 is shaped such that the distal opening 24 of the second through lumen 25 is recessed proximally relative to the distal opening 27 of the first through lumen 22. This allows the distal opening 24 fluidically couple with a proximal end 66 the air tube 3 but also exposes a distal end 67 of the air tube when the connector is coupled to the air tube. As illustrated in Figures 12 and 14, an underside of the lid section 63 incorporates a clamp 64 that is configured to snap-fit around the distal end 67 of the air tube 3 when the lid is closed. As illustrated in Figures 17 and 18, the annular flange 69 on the air tube 3 combined with the clamp 64 serves to lock the connector to the endoscope.

### Equivalents

The foregoing description details presently preferred embodiments of the present invention. The invention is defined in the following claims. Other embodiments, methods, examples etc. are not a part of the invention.

## Claims

1. A connector (20, 60) to fluidically connect a medical instrument (1) with an air tube and (34) a water tube (35), the connector comprising a housing (21) having:
a first through lumen (22) with a proximal opening (26) configured to couple with the air tube (34) and a distal opening (27) configured to couple with an air port (3) of the medical instrument; and
a second through lumen (25) with a proximal opening (23) configured to couple with a water tube (35) and a distal opening (24) configured to couple with a water port (2) of the medical instrument;
**characterised in that** the connector is a two-part connector comprising a proximal part (28) comprising the proximal openings (26, 23) and a distal part (29) comprising the distal openings (24, 27), wherein the proximal part and distal part are configured for detachable engagement, and wherein the second through lumen (25) comprises a non-return valve (10) disposed in the distal part of the housing.

2. A connector (20, 60) according to Claim 1, is which the distal openings (24, 27) are configured to fluidically connect with air and water ports (3, 2) of a scoping device.

3. A connector (20, 60) according to Claim 1, is which the distal openings (24, 27) are configured to fluidically connect with an air-water (AW) port (3, 2) of an endoscope.

4. A connector (20, 60) according to any preceding Claim, in which each proximal opening (23, 26) comprises a recessed socket dimensioned to receive an end of an air or water tube (34, 35).

5. A connector (20, 60) according to Claim 4, in which the recessed socket (26) of the first through lumen has a diameter greater than the recessed socket (23) of the second through lumen.

6. A connector (20, 60) as claimed in any preceding Claim, in which the proximal part (28) and distal part (29) are configured for detachable friction-fit engagement.

7. A connector (20, 60) according to any preceding Claim, in which a proximal end of the distal part (29) of the housing (31) comprises projecting ports (30), and a distal end of the proximal part (28) of the housing comprises recessed ports (31) configured to receive the projecting ports to fluidically couple the distal and proximal parts of the housing.

8. A connector (60) according to any preceding Claim, in which the proximal part (28) comprises a housing section (61) dimensioned to receive the distal part (29).

9. A connector (60) according to Claim 8, in which the housing section (61) of the proximal part (28) comprises a base section (62) and a lid section (63) hingedly mounted to the base section and adjustable from an open configuration to allow insertion of the distal part (29) into the housing section and a closed configuration in which the distal part (29) is enclosed within the housing section.

10. A connector (60) according to Claim 9, in which the lid section (63) comprises a clamp (64) configured to clamp the proximal part (28) of the housing (21) to the air port (3) or water port (2) of the medical instrument (1) when the lid section is in the closed configuration.

11. A connector (60) according to Claim 9, in which the lid section (63) comprises a clamp (64) configured to clamp the proximal part (28) of the housing (21) to the air port (3) of the medical instrument (1) when the lid section is in the closed configuration.

12. A connector (60) according to Claim 11, in which the distal opening (24) of the second through lumen (25) is recessed proximally relative to the distal opening (27) of the first through lumen (22).

13. An air and water tube assembly (33) comprising:
a connector (20, 60) according to any preceding Claim;
a water tube (35) having a distal end fluidically coupled to the second through lumen (25) via the proximal opening (23); and
an air tube (34) having a distal end fluidically coupled to the first through lumen (25) via the proximal opening (26).

14. An assembly according to Claim 13, in which the distal ends of the air and water tubes are non-detachably attached to the connector (20, 60).

15. An assembly according to Claim 13 or 14, including a bottle (36) and a bottle cap (37) non-detachably connected to a proximal part of the water tube (35) and air tube (34) to fluidically connect the bottle with the air and water tubes when the cap is attached to the bottle.

16. An assembly according to any of Claims 13 to 15, in which the water tube (35) has a bore that is smaller than a bore of the second tube.

17. A kit of parts comprising an assembly (33) of any of Claims 13 to 4 16 additionally including one or more replacement distal parts (29) of the connector (20) configured for detachable attachment to the proximal part (28) of the connector.

18. An adapter (50) for a connector (8) of the type that fluidically connects an air and water tube assembly with an air and water port (3, 2) of a medical instrument such as a scoping device (1), the adapter comprising a housing (51) having:
a distal end (52) with an air port socket (53) dimensioned to mate with the air port (3) of the medical instrument (1) and a water port socket (54) dimensioned to mate with the water port (2) of the medical instrument;
a proximal end (55) with an air port (56) and a water port (57) having the same configuration as the air and water ports (3, 2) of the medical instrument (1);
an air lumen fluidically connecting the air port socket (53) and the air port (56);
a water lumen fluidically connecting the water port socket (54) and water port (57); and
a non-return valve (10) disposed in the water lumen.

## Patentansprüche

1. Verbindungsstück (20, 60) zum Fluidverbinden eines medizinischen Instruments (1) mit einem Luftschlauch (34) und einem Wasserschlauch (35), wobei das Verbindungsstück ein Gehäuse (21) umfasst, das Folgendes aufweist:
ein erstes durchgehendes Lumen (22) mit einer proximalen Öffnung (26), die so ausgelegt ist, dass sie sich an den Luftschlauch (34) anschließen lässt, und einer distalen Öffnung (27), die so ausgelegt ist, dass sie sich an einen Luftanschluss (3) des medizinischen Instruments anschließen lässt, und
ein zweites durchgehendes Lumen (25) mit einer proximalen Öffnung (23), die so ausgelegt ist, dass sie sich an einen Wasserschlauch (35) anschließen lässt, und einer distalen Öffnung (24), die so ausgelegt ist, dass sie sich an einen Wasseranschluss (2) des medizinischen Instruments anschließen lässt,
**dadurch gekennzeichnet, dass** es sich bei dem Verbindungsstück um ein zweiteiliges Verbindungsstück handelt, das einen proximalen Teil (28) mit den proximalen Öffnungen (26, 23) und einen distalen Teil (29) mit den distalen Öffnungen (24, 27) umfasst, wobei der proximale und der distale Teil für eine lösbare formschlüssige Verbindung ausgelegt sind und das zweite durchgehende Lumen (25) ein Rückschlagventil (10) umfasst, das in dem distalen Teil des Gehäuses angeordnet ist.

2. Verbindungsstück (20, 60) nach Anspruch 1, bei dem die distalen Öffnungen (24, 27) so ausgelegt sind, dass sie sich mit Luft- und Wasseranschluss (3, 2) eines Sondiergeräts fluidverbinden lassen.

3. Verbindungsstück (20, 60) nach Anspruch 1, bei dem die distalen Öffnungen (24, 27) so ausgelegt sind, dass sie sich mit einem Luft-Wasser-(LW)-Anschluss (3, 2) eines Endoskops fluidverbinden lassen.

4. Verbindungsstück (20, 60) nach einem der vorhergehenden Ansprüche, bei dem jede proximale Öffnung (23, 26) eine ausgesparte Buchse umfasst, die so bemessen ist, dass sie ein Ende eines Luft- oder Wasserschlauchs (34, 35) aufnehmen kann.

5. Verbindungsstück (20, 60) nach Anspruch 4, bei dem die ausgesparte Buchse (26) des ersten durchgehenden Lumens einen größeren Durchmesser aufweist als die ausgesparte Buchse (23) des zweiten durchgehenden Lumens.

6. Verbindungsstück (20, 60) nach einem der vorhergehenden Ansprüche, bei dem der proximale Teil (28) und der distale Teil (29) für eine lösbare reibschlüssige Verbindung ausgelegt sind.

7. Verbindungsstück (20, 60) nach einem der vorhergehenden Ansprüche, bei dem ein proximales Ende des distalen Teils (29) des Gehäuses (31) vorstehende Anschlüsse (30) umfasst und ein distales Ende des proximalen Teils (28) des Gehäuses ausgesparte Anschlüsse (31) umfasst, die so ausgelegt sind, dass sie die vorstehenden Anschlüsse aufnehmen können und so den distalen und den proximalen Teil des Gehäuses fluidkoppeln.

8. Verbindungsstück (60) nach einem der vorhergehenden Ansprüche, bei dem der proximale Teil (28) einen Gehäuseabschnitt (61) umfasst, der so bemessen ist, dass er den distalen Teil (29) aufnehmen kann.

9. Verbindungsstück (60) nach Anspruch 8, bei dem der Gehäuseabschnitt (61) des proximalen Teils (28) einen Grundabschnitt (62) umfasst und einen Deckelabschnitt (63), der an den Grundabschnitt angelenkt und aus einer offenen Anordnung, die ein Einführen des distalen Teils (29) in den Gehäuseabschnitt ermöglicht, und einer geschlossenen Anordnung, in der der distale Teil (29) von dem Gehäuseabschnitt umschlossen ist, verstellbar ist.

10. Verbindungsstück (60) nach Anspruch 9, bei dem der Deckelabschnitt (63) eine Klemme (64) umfasst, die so ausgelegt ist, dass sie den proximalen Teil (28) des Gehäuses (21) an dem Luftanschluss (3) oder dem Wasseranschluss (2) des medizinischen Instruments (1) festklemmt, wenn sich der Deckelabschnitt in der geschlossenen Anordnung befindet.

11. Verbindungsstück (60) nach Anspruch 9, bei dem der Deckelabschnitt (63) eine Klemme (64) umfasst, die so ausgelegt ist, dass sie den proximalen Teil (28) des Gehäuses (21) an dem Luftanschluss (3) des medizinischen Instruments (1) festklemmt, wenn sich der Deckelabschnitt in der geschlossenen Anordnung befindet.

12. Verbindungsstück (60) nach Anspruch 11, bei dem die distale Öffnung (24) des zweiten durchgehenden Lumens (25) in Bezug auf die distale Öffnung (27) des ersten durchgehenden Lumens (22) proximal ausgespart ist.

13. Luft- und Wasserschlauchbaugruppe (33), die Folgendes umfasst:
ein Verbindungsstück (20, 60) nach einem der vorhergehenden Ansprüche,
einen Wasserschlauch (35) mit einem distalen Ende, das über die proximale Öffnung (23) mit dem zweiten durchgehenden Lumen (25) fluidgekoppelt ist, und
einen Luftschlauch (34) mit einem distalen Ende, das über die proximale Öffnung (26) mit dem ersten durchgehenden Lumen (25) fluidgekoppelt ist.

14. Baugruppe nach Anspruch 13, bei der die distalen Enden des Luft- und des Wasserschlauchs nicht lösbar an dem Verbindungsstück (20, 60) befestigt sind.

15. Baugruppe nach Anspruch 13 oder 14, die eine Flasche (36) umfasst und eine Flaschenkappe (37), die nicht lösbar mit einem proximalen Teil des Wasserschlauchs (35) und des Luftschlauchs (34) verbunden ist und die Flasche so mit dem Luft-und dem Wasserschlauch fluidverbindet, wenn die Kappe an der Flasche befestigt ist.

16. Baugruppe nach einem der Ansprüche 13 bis 15, bei der der Wasserschlauch (35) eine Bohrung aufweist, die kleiner ist als eine Bohrung des zweiten Schlauchs.

17. Bausatz aus Teilen mit einer Baugruppe (33) nach einem der Ansprüche 13 bis 16, der zusätzlich ein oder mehrere distale Ersatzteile (29) für das Verbindungsstück (20) aufweist, die für ein lösbares Befestigen an dem proximalen Teil (28) des Verbindungsstücks ausgelegt sind.

18. Adapter (50) für ein Verbindungsstück (8) von dem Typ, der eine Luft- und Wasserschlauchbaugruppe mit einem Luft- und Wasseranschluss (3, 2) eines medizinischen Instruments wie eines Sondiergeräts (1) fluidverbindet, wobei der Adapter ein Gehäuse (51) umfasst, das Folgendes aufweist:
ein distales Ende (52) mit einer Luftanschlussbuchse (53), die so bemessen ist, dass sie zu dem Luftanschluss (3) des medizinischen Instruments (1) passt, und einer Wasseranschlussbuchse (54), die so bemessen ist, dass sie zu dem Wasseranschluss (2) des medizinischen Instruments passt,
ein proximales Ende (55) mit einem Luftanschluss (56) und einem Wasseranschluss (57) mit der gleichen Anordnung wie der Luft- und der Wasseranschluss (3, 2) des medizinischen Instruments (1),
ein Luftlumen, das die Luftanschlussbuchse (53) und den Luftanschluss (56) fluidverbindet, ein Wasserlumen, das die Wasseranschlussbuchse (54) und den Wasseranschluss (57) fluidverbindet, und
ein Rückschlagventil (10), das in dem Wasserlumen angeordnet ist.

## Revendications

1. Connecteur (20, 60) pour connecter de manière fluidique un instrument médical (1) avec un tube d'air (34) et un tube d'eau (35), le connecteur comprenant un logement (21) ayant :
une première lumière traversante (22) avec une ouverture proximale (26) configurée pour se coupler avec le tube d'air (34) et une ouverture distale (27) configurée pour se coupler avec un orifice d'air (3) de l'instrument médical ; et
une deuxième lumière traversante (25) avec une ouverture proximale (23) configurée pour se coupler avec un tube d'eau (35) et une ouverture distale (24) configurée pour se coupler avec un orifice d'eau (2) de l'instrument médical ;
**caractérisé en ce que** le connecteur est un connecteur en deux parties comprenant une partie proximale (28) comprenant les ouvertures proximales (26, 23) et une partie distale (29) comprenant les ouvertures distales (24, 27), dans lequel la partie proximale et la partie distale sont configurées pour une entrée en prise détachable, et dans lequel la deuxième lumière traversante (25) comprend un clapet anti-retour (10) disposé dans la partie distale du logement.

2. Connecteur (20, 60) selon la revendication 1, dans lequel les ouvertures distales (24, 27) sont configurées pour se connecter de manière fluidique avec des orifices d'air et d'eau (3, 2) d'un dispositif d'endoscopie.

3. Connecteur (20, 60) selon la revendication 1, dans lequel les ouvertures distales (24, 27) sont configurées pour se connecter de manière fluidique avec un orifice d'air-eau (AE) (3, 2) d'un endoscope.

4. Connecteur (20, 60) selon l'une quelconque des revendications précédentes, dans lequel chaque ouverture proximale (23, 26) comprend une emboîture renfoncée dimensionnée pour recevoir une extrémité d'un tube d'air ou d'eau (34, 35).

5. Connecteur (20, 60) selon la revendication 4, dans lequel l'emboîture renfoncée (26) de la première lumière traversante a un diamètre supérieur à l'emboîture renfoncée (23) de la deuxième lumière traversante.

6. Connecteur (20, 60) selon l'une quelconque des revendications précédentes, dans lequel la partie proximale (28) et la partie distale (29) sont configurées pour une entrée en prise par ajustement serré détachable.

7. Connecteur (20, 60) selon l'une quelconque des revendications précédentes, dans lequel une extrémité proximale de la partie distale (29) du logement (31) comprend des orifices saillants (30), et une extrémité distale de la partie proximale (28) du logement comprend des orifices renfoncés (31) configurés pour recevoir les orifices saillants pour coupler de manière fluidique les parties distale et proximale du logement.

8. Connecteur (60) selon l'une quelconque des revendications précédentes, dans lequel la partie proximale (28) comprend une section de logement (61) dimensionnée pour recevoir la partie distale (29).

9. Connecteur (60) selon la revendication 8, dans lequel la section de logement (61) de la partie proximale (28) comprend une section de base (62) et une section de couvercle (63) montée de manière articulée sur la section de base et ajustable depuis une configuration ouverte pour permettre l'insertion de la partie distale (29) jusque dans la section de logement et une configuration fermée dans lequel la partie distale (29) est enfermée au sein de la section de logement.

10. Connecteur (60) selon la revendication 9, dans lequel la section de couvercle (63) comprend une bride de serrage (64) configurée pour serrer la partie proximale (28) du logement (21) avec l'orifice d'air (3) ou l'orifice d'eau (2) de l'instrument médical (1) quand la section de couvercle est dans la configuration fermée.

11. Connecteur (60) selon la revendication 9, dans lequel la section de couvercle (63) comprend une bride de serrage (64) configurée pour serrer la partie proximale (28) du logement (21) avec l'orifice d'air (3) de l'instrument médical (1) quand la section de couvercle est dans la configuration fermée.

12. Connecteur (60) selon la revendication 11, dans lequel l'ouverture distale (24) de la deuxième lumière traversante (25) est renfoncée de manière proximale par rapport à l'ouverture distale (27) de la première lumière traversante (22).

13. Ensemble de tubes d'air et d'eau (33) comprenant :
un connecteur (20, 60) selon l'une quelconque des revendications précédentes ;
un tube d'eau (35) ayant une extrémité distale couplée de manière fluidique à la deuxième lumière traversante (25) via l'ouverture proximale (23) ; et
un tube d'air (34) ayant une extrémité distale couplée de manière fluidique à la première lumière traversante (25) via l'ouverture proximale (26).

14. Ensemble selon la revendication 13, dans lequel les extrémités distales des tubes d'air et d'eau sont attachées de manière non détachable au connecteur (20, 60).

15. Ensemble selon la revendication 13 ou 14, incluant une bouteille (36) et un bouchon de bouteille (37) connecté de manière non détachable à une partie proximale du tube d'eau (35) et du tube d'air (34) pour connecter de manière fluidique la bouteille avec les tubes d'air et d'eau quand le bouchon est attaché à la bouteille.

16. Ensemble selon l'une quelconque des revendications 13 à 15, dans lequel le tube d'eau (35) a un alésage qui est plus petit qu'un alésage du deuxième tube.

17. Trousse de pièces comprenant un ensemble (33) selon l'une quelconque des revendications 13 à 16, incluant en plus une ou plusieurs parties distales de remplacement (29) du connecteur (20) configurées pour être attachées de manière détachable à la partie proximale (28) du connecteur.

18. Adaptateur (50) pour un connecteur (8) du type qui connecte de manière fluidique un ensemble de tubes d'air et d'eau avec un orifice d'air et d'eau (3, 2) d'un instrument médical tel qu'un dispositif d'endoscopie (1), l'adaptateur comprenant un logement (51) ayant :
une extrémité distale (52) avec une emboîture d'orifice d'air (53) dimensionnée pour s'accoupler avec l'orifice d'air (3) de l'instrument médical (1) et une emboîture d'orifice d'eau (54) dimensionnée pour s'accoupler avec l'orifice d'eau (2) de l'instrument médical ;
une extrémité proximale (55) avec un orifice d'air (56) et un orifice d'eau (57) ayant la même configuration que les orifices d'air et d'eau (3, 2) de l'instrument médical (1) ;
une lumière d'air connectant de manière fluidique l'emboîture d'orifice d'air (53) et l'orifice d'air (56) ;
une lumière d'eau connectant de manière fluidique l'emboîture d'orifice d'eau (54) et l'orifice d'eau (57) ; et
un clapet anti-retour (10) disposé dans la lumière d'eau.
